# EUROPEAN PATENT SPECIFICATION

(11) **EP 2 968 606 B1**
(45) Date of publication and mention of the grant of the patent: **07.10.2020**
(21) Application number: 14764420.7
(22) Date of filing: 14.03.2014
(51) Int. Cl.: A61K 48/00, A61K 8/64, A61P 17/14, A61Q 7/00, C12N 15/85

(54) **TREATMENT OF AUTOIMMUNE DISEASES**
BEHANDLUNG VON AUTOIMMUNERKRANKUNGEN
TRAITEMENT DE MALADIES AUTO-IMMUNES

(30) Priority: 15.03.2013 US 201361800354 P
(43) Date of publication of application: 20.01.2016
(73) Proprietor: Loma Linda University, Loma Linda, California 92350 (US)
(72) Inventor: ESCHER, Alan P., Redlands, California 92374 (US)
(74) Representative: V.O.
(86) International application number: PCT/US2014/029685
(87) International publication number: WO 2014/145042

(56) References cited:
- EP-B1- 2 058 393
- WO-A1-2009/114735
- WO-A2-2006/124375
- WO-A2-2013/044177
- CHAOPATCHAYAKUL, P. ET AL.: 'Aberrant DNA methylation of apoptotic signaling genes in patients responsive and nonresponsive to therapy for cervical carcinoma' AMERICAN JOURNAL OF OBSTETRICS AND GYNECOLOGY vol. 202, no. 3, 2010, pages 281.E1 - 281.E9, XP026942892 DOI: 10.1016/J.AJOG.2009.11.037
- PEZZICOLI, A. ET AL.: 'Expression in T-cells of the proapoptotic protein p66SHC is controlled by promoter demethylation' BIOCHEMICAL AND BIOPHYSICAL RESEARCH COMMUNICATIONS vol. 349, no. 1, 13 October 2006, pages 322 - 328, XP027154446 DOI: 10.1016/J.BBRC.2006.08.039
- YI, A.-K. ET AL.: 'CpG motifs in bacterial DNA activate leukocytes through the pH-dependent generation of reactive oxygen species' THE JOURNAL OF IMMUNOLOGY vol. 160, no. 10, 15 May 1998, pages 4755 - 4761, XP002979516

## Description

### RELATED APPLICATION

This application claims the benefit of U.S. Patent Application No. 2014/288,164 filed on March 17, 2014 claiming priority of March 15, 2013.

### FIELD

The subject technology relates generally to a polynucleotide and its use in a method for the treatment of autoimmune diseases or conditions, in particular autoimmune diseases or conditions that result in hair loss, such as alopecia.

### BACKGROUND

Alopecia is a condition in which hair is lost. The loss of hair in alopecia is not limited just to head hair but can happen anywhere on the body. Although not usually life-threatening, since it is accompanied by serious emotional distress due to issues related to appearance, there is a desire for an excellent agent for the treatment and an excellent agent for the prevention of alopecia. Furthermore, since alopecia is often accompanied by fading of hair color, there is a desire for an agent for the prevention and an agent for the treatment of fading of hair color accompanying alopecia. Moreover, since alopecia is often accompanied by deterioration of hair quality such as hair becoming finer or hair becoming shorter, there is a desire for an agent for the prevention and an agent for the treatment of deterioration of hair quality accompanying alopecia.

With regard to types of alopecia, there are alopecia areata, androgenetic alopecia, postmenopausal alopecia, female pattern alopecia, seborrheic alopecia, alopecia pityroides, senile alopecia, cancer chemotherapy drug-induced alopecia, alopecia due to radiation exposure, trichotillomania, postpartum alopecia, etc.

These types of alopecia have the same symptoms of hair loss, but are based on different causes, and therapies therefor are different from each other. In particular, androgenetic alopecia, which is based on the action of male hormone, and alopecia areata, which is suspected to be an immune disease, are very different diseases. Furthermore, it is thought that postpartum alopecia, female pattern alopecia, seborrheic alopecia, alopecia pityroides, senile alopecia, cancer chemotherapy drug-induced alopecia, and alopecia due to radiation exposure have different causes from each other, and there are hardly any effective therapies.

Alopecia areata is alopecia in which coin-sized circular to patchy bald area(s) with a clear outline suddenly occur, without any subjective symptoms or prodromal symptoms, etc. in many cases, and subsequently when spontaneous recovery does not occur they gradually increase in area and become intractable. Alopecia areata is suspected to be an autoimmune disease, but the cause thereof has not yet been discovered, and there is no known definite treatment method.

Alopecia areata is known to be associated with an autoimmune disease such as a thyroid disease represented by Hashimoto's disease, vitiligo, systemic lupus erythematosus, rheumatoid arthritis, or myasthenia gravis or an atopic disease such as bronchial asthma, atopic dermatitis, or allergic rhinitis.

Androgenetic alopecia (AGA) is alopecia in which male hormone acts on male hormone-sensitive hair follicles to form vellus hair, and occurs in about half of males and 10% to 20% of females. It is thought that genetic predisposition is a large factor in androgenetic alopecia; in androgenetic alopecia in males, the head hair on the frontal region and crown becomes fine and short and turns into vellus hair, the hair line on the forehead finally retreats, and head hair on the crown is lost. On the other hand, in androgenetic alopecia in females, in general the hairline does not change but hair of the entire head, in particular the crown and the frontal region, becomes fine. Finasteride improves only about ¼ of patients for androgenetic alopecia in males, and since administration of finasteride to females is contraindicated, finasteride cannot be used for androgenetic alopecia in females.

Postpartum alopecia is alopecia in which hair whose growth phase has been maintained by estrogen enters the resting phase all at once due to childbirth, and hair loss increases. The hair loss of postpartum alopecia usually starts approximately 2 months after childbirth and continues until about 6 months after childbirth; since it usually recovers within 1 year unless there is late childbearing, in most cases a treatment is not particularly required, but there are cases in which hair does not recover spontaneously.

Female pattern alopecia is alopecia that is thought to occur due to a decrease in the amount of the female hormone estrogen relative to the amount of androgen in the bloodstream. It often occurs after the menopause, and in this case it is also called postmenopausal alopecia. Female pattern alopecia might be improved by hormone replacement therapy but is intractable in many cases.

Seborrheic alopecia is alopecia that is caused by excessive sebum secretion on the scalp, pores are blocked thereby causing inflammation around the pores or in the hair root, and the hair falls out. Seborrheic alopecia is improved to some extent by removing sebum by washing the hair, but it easily reoccurs and exhibits intractability.

Alopecia pityroides is alopecia that is caused by dandruff blocking pores to thus cause inflammation. Alopecia pityroides is often caused by excessive hair washing; remission is achieved by reducing the number of times of hair washing or using a shampoo having a weak washing power, but it easily reoccurs and is intractable.

Trichotillomania is alopecia due to a hair-pulling disorder. Trichotillomania is a symptom resulting from pathological anxiety and can be treated by behavioral therapy or psychological therapy.

Senile alopecia is alopecia in which, due to aging regardless of gender, body hair of the entire body, including all of the head hair, gradually becomes thinner. It is thought that this is a natural phenomenon that appears in many people due to aging, and this is not particularly a target for treatment at the present. However, there is a desire for improvement since there is an increased social requirement for improving the quality of life of elderly people accompanying a rise in the average lifespan.

Cancer chemotherapy drug-induced alopecia is alopecia that is a side effect of anticancer treatment with a cancer chemotherapy drug. The shock to the patient caused by loss of hair in all areas including not only the head but also the eyebrows, eyelashes, nasal hair, underarm hair, and pubic hair is profound even if it is explained in advance. Since this also hinders the carrying out of cancer chemotherapy, there is a high need for a treatment for this. Similarly, alopecia accompanying radiation exposure is also alopecia that occurs based on the same mechanism as that for cancer chemotherapy drug-induced alopecia in terms of cancer cells being selectively killed by inhibiting cell division. Therefore, a drug that can treat alopecia accompanying cancer chemotherapy can also treat alopecia accompanying radiation exposure.

In addition, alopecia due to an adverse reaction to a drug such as an antithyroid drug, an anticoagulant, thallium, a psychotropic drug, or a β-blocker, alopecia due to a fungus, alopecia due to an endocrine disorder such as dyspituitarism, hypothyroidism, or hyperthyroidism, alopecia due to a metabolic disorder such as a nutritional disorder, hypoalbuminemia, cachexia, iron-deficiency anemia, zinc deficiency, homocystinuria, or cirrhosis of the liver, toxic alopecia, alopecia due to high temperature, childbirth, major surgery, sudden body weight loss, or serious illness, etc. are known, and they can be tackled by removing the respective causes thereof.

Among these types of alopecia, female pattern alopecia, seborrheic alopecia, and alopecia pityroides can be tackled to some extent by removing the respective causes thereof, but they easily reoccur, and are intractable. Furthermore, although it is thought that the cause of female pattern alopecia is related to hormone balance, hormone replacement therapy is indicated for menopausal disorders, osteoporosis, and hyperlipidemia but is not indicated for female pattern alopecia; since there is a possibility of cancer being caused by hormone replacement therapy, hormone replacement therapy is not carried out for the purpose of treating female pattern alopecia. Furthermore, with regard to androgenetic alopecia, there is no adequate therapy yet, and with regard to alopecia areata, even the cause thereof is little understood.

As described above, among the types of alopecia, the types of alopecia that are difficult to treat are alopecia areata and androgenetic alopecia, and for alopecia areata in particular there are hardly any effective treatment methods. Moreover, there are hardly any therapies for postpartum alopecia, female pattern alopecia, alopecia pityroides, senile alopecia, and cancer chemotherapy drug-induced alopecia.

There is a need to develop therapeutic agents to treat hair loss.

### SUMMARY

The subject technology generally relates to a polynucleotide and its use in methods to treat hair loss or to stimulate the growth of hair.

### DETAILED DESCRIPTION

### 1. OVERVIEW

The subject technology generally relates to a polynucleotide and its use in methods to treat hair loss or to stimulate the growth of hair.

As described and exemplified herein, hair loss, such as alopecia areata, is a condition often attributable to an autoimmune disease that targets hair follicles. By delivering a pro-apoptotic protein (e.g., to the site of inflammation or the site of hair loss), tolerogenic apoptosis of hair follicles is induced by the pro-apoptotic protein. As a result, the apoptotic follicles containing autoantigen(s) are produced, which in turn induce a regulatory response specific for hair follicles. The regulatory response induces immune tolerance to autoantigen(s), thereby reducing the abnormal autoimmune response that attacks hair follicle cells.

### 2. DEFINITIONS

The use of numerical values in the various ranges specified in this application, unless expressly indicated otherwise, are stated as approximations as though the minimum and maximum values within the stated ranges were both preceded by the word "about." Slight variations above and below the stated ranges may be used to achieve substantially the same results as values within the ranges. The disclosure of ranges is intended as a continuous range including every value between the minimum and maximum values recited as well as any ranges that can be formed by such values. Accordingly, the skilled person will appreciate that many such ratios, ranges, and ranges of ratios can be unambiguously derived from the data and numbers presented herein and all represent various embodiments of the subject technology.

As used in this disclosure, the term "autoantigen" means and includes an endogenous antigen that stimulates the production of autoantibodies, as in an autoimmune reaction, as well as part of such endogenous antigens, or modified endogenous antigens that elicit the same response as the full endogenous antigen, as will be understood by those with skill in the art with reference to this disclosure. For example, in the context of this disclosure carbonic anhydrase II, chromogranin, collagen, CYP2D6 (cytochrome P450, family 2, subfamily Device 400, polypeptide 6), glutamic acid decarboxylase, secreted glutamic acid decarboxylase 55, hCDR1, HSP60, IA2, IGRP, insulin, myelin basic protein, hNinein, Ro 60kDa, SOX-10 (SRY-box containing gene 10), ZnT8, and the like, are autoantigens. Also encompassed are antigenic fragments of the any one of the foregoing autoantigens.

The term "about", as used here, refers to +/- 5% of a value.

The term "functional fragment" of protein refers to refers to a peptide fragment that is a portion of the full length protein, and has substantially the same biological activity, or carries out substantially the same function as the full length protein (e.g., carrying out the same enzymatic reaction). For example, a functional fragment of a pro-apoptotic protein can promote the apoptosis of a cell.

The term "hypermethylated" (sometimes abbreviated as "methylated") when used in reference to a DNA, means that at least about 30% (preferably at least about 35%, or at least about 40%, or at least about 45%, at least about 50%, at least about 55%, at least about 60%, at least about 65%, at least about 70%) of the CpG dinucleotides are methylated. For example, mammalian DNA, in which between 60% and 90% of all CpGs are methylated, is hypermethylated.

The term "hypomethylated" (sometimes abbreviated as "unmethylated") when used in reference to a DNA, means that about 15% or less (preferably about 10% or less, about 7.5% or less, about 5% or less, or about 3%, or about 1% or less) of the CpG dinucleotides are methylated. For example, bacterial DNA, in which between 5% and 15% of all CpGs are methylated, is hypomethylated.

A "methylation" level, when used in reference to a DNA, refers to the percentage of methylated CpG dinucleotides out of the total CpG dinucleotides of the DNA molecule.

The term "controlled" means "operably linked," which refers to the association of a nucleic acid sequence on another nucleic acid fragment so that the function of one is affected by the other. For example, a coding sequence is "controlled" by a promoter when the promoter is capable of affecting the expression of that coding sequence (i.e., that the coding sequence is under the transcriptional control of the promoter). Coding sequences can be operably linked to regulatory sequences in sense or antisense orientation.

### 3. POLYNUCLEOTIDES ENCODING A PRO-APOPTOTIC PROTEIN

In one aspect, the invention provides a polynucleotide that comprises a sequence that encodes a pro-apoptotic protein, or a functional fragment thereof, and wherein about 30% to about 60% of the CpG dinucleotides of the polynucleotide are methylated.

In another aspect, the invention provides a polynucleotide that comprises a sequence that encodes a pro-apoptotic protein, or a functional fragment thereof, and wherein the methylation level of CpG dinucleotides of the polynucleotide is about 2 fold to about 4 fold higher as compared to the average methylation level of CpG dinucleotides in a wild type Escherichia coli *(E. coli)* genome.

### A. Methylation Levels

DNA methylation in vertebrates typically occurs at CpG sites. This methylation results in the conversion of the cytosine to 5-methylcytosine. The formation of Me-CpG is catalyzed by the enzyme DNA methyltransferase. Between 60% and 90% of all CpGs are methylated in mammals. Human DNA has about 80%-90% of CpG sites methylated.

Bacterial DNA contains low levels of methylated CpG dinucleotides compared to mammalian DNA. The mammalian immune system uses this feature of bacterial DNA as a signal to identify foreign DNA and respond to threats by bacterial pathogens.

Therefore, the polynucleotide described herein is preferably hypermethylated to avoid being recognized as a bacterial pathogen when administered to a mammalian subject. For example, at least about 70%, at least about 65%, at least about 60%, at least about 55%, at least about 50%, at least about 45% , at least about 40% , at least about 35% , at least about 30%, from about 30% to about 70%, from about 30% to about 65%, from about 30% to about 60%, from about 30% to about 55%, from about 30% to about 50%, from about 30% to about 45%, from about 30% to about 40%, from about 25% to about 60%, from about 25% to about 55%, from about 25% to about 50%, of the CpG dinucleotides of the polynucleotide are methylated.

Alternatively, the methylation level of the polynucleotide can be determined by comparing the overall CpG dinucleotides methylation level against a baseline. A suitable baseline methylation level is the average CpG dinucleotides methylation level of a bacterial DNA (e.g., bacterial genome) from a wild type bacterium (e.g., an Escherichia coli *(E. coli).* For example, methylation level of CpG dinucleotides of the polynucleotide can be at least at least about 2 fold, at least about 2.5 fold, at least about 3 fold, at least about 3.5 fold, at least about 4 fold, or from about 2 fold to about 4 fold, higher as compared to the average methylation level of CpG dinucleotides in a wild type Escherichia coli (*E. coli*) genome, or the average methylation level of CpG dinucleotides in a plasmid from a wild type E. coli. The average methylation level of CpG dinucleotides in wild type bacterial strains are generally known or ascertainable.

The methylation level of the polynucleotide described herein, or the baseline average methylation level of wild type bacterium can also be determined using art known methods. For example, WO2011109529 discloses methods for detecting the prevalence of methylation of a DNA sequence in a DNA sample; see also, Wu et al., Statistical Quantification of Methylation Levels by Next-Generation Sequencing, PLoS ONE 6(6): e21034. doi:10.1371/journal.pone.0021034.

Methods of making a polynyucleotide with a desired methylation level can be achieved by multiple methods. For example, PCT Patent Application PCT/US12/56761 discloses a bacterial strain comprising an engineered polynucleotide sequence encoding a methylase controlled by a constitutive promoter, wherein said engineered polynucleotide is stably incorporated into the chromosomal DNA of said bacterium. This bacterial strain can be used to produce polynucleotides that have a desired methylation level. For example, levels of methylation can be adjusted by modulating the expression level of methylase in the bacterial host (for example, by using promoters of different strength). In vitro methylation may also be used. See, e.g., Wyngaert et al., Genomic Imprinting: Methods and Protocols, at Chapter 18, In Vitro Methylation of Predetermined Regions in Recombinant DNA Constructs 243-250, DOI: 10.1385/1-59259-211-2:243.

### B. Pro-apoptotic proteins

Proapoptotic proteins refers to proteins that are able to activate directly or indirectly the mechanisms of cell death by apoptosis.

Suitable pro-apoptotic proteins include, e.g., Bax, Bak, Bim, Puma, Bad, Bik, Noxa, Bmf, Hrk, Bid, FAS, a caspase mutant (a modified caspase), a survivin mutant (a modified survivin protein), Death Receptor 4 (DR4), Death Receptor 5 (DR5), a FAS receptor, and a Tumor Necrosis Factor Receptor. A functional fragment of any one of these pro-apoptotic proteins is also encompassed.

Exemplary pro-apoptotic proteins described herein have been characterized.

Members of the Bcl-2 family represent some of the most well-defined regulators of apoptosis pathway. Some members of the Bcl-2 family, including Bcl-2, Bcl-XL, Ced-9, Bcl-w and so forth, promote cell survival, while other members including Bax, Bcl-Xs, Bad, Bak, Bid, Bik and Bim have been shown to potentiate apoptosis (Adams and Cory, 1998). Biological functions of the Bcl-2 family members include dimer formation (Oltvai et al., J. Bcl-2 heterodimerizes in vivo with a conserved homolog, Bax, that accelerates programmed cell death. Cell, 74: 609-619, 1993.), protease activation (Chinnaiyan et al., a novel FAD D-homologous ICE/CED-3-like protease, is recruited to the CD95 (Fas/APO-1) death-inducing signaling complex. Cell 1996; 85: 817-827), mitochondrial membrane depolarization , generation of reactive oxygen intermediates (Hockenbery et al., Bcl-2 functions in an antioxidant pathway to prevent apoptosis. Cell, 75: 241-251, 1993), regulation of calcium flux (Lam et al., Evidence that Bcl-2 represses apoptosis by regulating endoplasmic reticulum-associated Ca2+ fluxes. Proc. Natl. Acad. Sci. USA, 91: 6569-6573, 1994; Huiling et al., Maintenance of calcium homeostasis in the endoplasmic reticulum by Bcl-2. J. Cell Biol. 138: 1219-1228, 1997), and pore formation.

In the Bcl-2 family there is notable homology clustered within two conserved regions: Bcl-2 homology domains 1 and 2 (BH1 and BH2) (Oltvai et al., 1993; Boise et al., bcl-x, a bcl-2-related gene that functions as a dominant regulator of apoptotic cell death. Cell. 1993 Aug. 27; 74(4):597-608; Kozopas et al., MCL1, a gene expressed in programmed myeloid cell differentiation, has sequence similarity to BCL2. Proc Natl Acad Sci USA. 1993 Apr. 15; 90(8):3516-20; Lin et al., Characterization of Al, a novel hemopoietic-specific early-response gene with sequence similarity to bcl-2. J. Immunol. 1993 Aug. 15; 151(4):1979-88). Another conserved domain in Bax, distinct from BH1 and BH2, is termed BH3 and mediates cell death and protein binding functions. A subset of the pro-apoptotic proteins contains only the BH3 domain, implying that this particular domain may be uniquely important in the promotion of apoptosis (Diaz et al., Dimerization properties of human BAD. Identification of a B H-3 domain and analysis of its binding to mutant BCL-2 and BCL-XL proteins. J Biol. Chem. 1997 Dec. 5; 272(49):30866-72).

Bax, a 21 kDa death-promoting member of the Bcl-2 family, was first identified as a protein that co-immunoprecipated with Bcl-2 from different cell lines (Oltvai et al., 1993). Overexpression of Bax accelerates cell death in response to a wide range of cytotoxic results. Determination of the amino acid sequence of the Bax protein showed it to be highly homologous to Bcl-2. The Bax gene consists of six exons and produces alternative transcripts, the predominant form of which encodes a 1.0 kb mRNA and is designated Baxa. Like Bcl-2 and several other members of the Bcl-2 family, the Bax protein has highly conserved regions, BH1, BH2 and BH3 domains, and hydropathy analysis of the sequences of these proteins indicates the presence of a hydrophobic transmembrane segment at their C-terminal ends (Oltvai et al., 1993). Bax is widely expressed without any apparent tissue specificity.

A splice variant of Bax, Bax-alpha, has also been reported.

Bak is expressed in a wide variety of cell types and binds to the Bcl-2 homologue Bcl-x2 in yeast. A domain in Bak was identified as both necessary and sufficient for cytotoxicity activity and binding to Bcl-x1. Furthermore, sequences similar to this domain that are distinct from BH1 and BH2 have been identified in Bax and Bipl. This domain is critical for mediating the function of multiple cell death-regulatory proteins that interact with Bcl-2 family members.

Bad possesses the key amino acid motifs of BH1 and BH2 domains. Bad lacks the classical C-terminal signal-anchor sequence responsible for the integral membrane positions of other family members. Bad selectively dimerizes with Bcl-X_{L} as well as Bcl-2, but not with Bax, BcI-Xs-Mcl1, A1 or itself. Bad reverses the death repressor activity of Bcl-X_{L}, but not that of Bcl-2.

Bik, another member of the Bcl-2 family, interacts with the cellular survival-promoting proteins, Bcl-2 and Bcl-X_{L} as well as the viral survival-promoting proteins, Epstein Barr virus-BHRFl and adenovirus E1B-19 kDa. In transient transfection assays, Bik promotes cell death in a manner similar to Bax and Bak, other pro-apoptotic members of the Bcl-2 family. This pro-apoptosis activity of Bik can be suppressed by coexpression of Bcl-2, Bcl-X_{L}, EBV-BHRF1 and EIB-19 kDa proteins, which suggests that Bik may be a common target for both cellular and viral anti-apoptotic proteins. While Bik does not contain overt homology to the BH1 and BH2 conserved domains characteristic of the Bcl-2 family, it shares a 9 amino acid domain (BH3) with Bax and Bak, which may be a critical determinant for the death-promoting activity of these proteins.

PUMA (p53 upregulated modulator of apoptosis) is a target for activation by p53. A gene encodes two BH3 domain-containing proteins (PUMA-alpha and PUMA-beta) that are induced in cells following p53 activation. PUMA-alpha and PUMA-beta show similar activities; they bind to Bcl-2, localize to the mitochondria to induce cytochrome c release, and activate the rapid induction of programmed cell death. Antisense inhibition of PUMA expression reduced the apoptotic response to p53, and PUMA is likely to play a role in mediating p53-induced cell death through the cytochrome c/Apaf-1-dependent pathway. See, e.g., Nakano et al., Mol Cell. 2001 Mar;7(3):683-94.

The BH3-only protein Bim is a pro-apoptotic protein that induces Bax/Bak-oligomerization on mitochondria. See, e.g., Gogada et al., BIM, a proapoptotic protein, upregulated via transcription factor E2F1-dependent mechanism, functions as a prosurvival molecule in cancer, doi:10.1074/jbc.M112.386102jbc.M112.386102.

Activation of the intrinsic apoptotic pathway by p53 often requires the transcription of the proapoptotic Bcl-2 proteins Noxa, Puma, or both. It has been reported that Noxa is required for particulate matter-induced cell death and lung inflammation.

Bmf is a proapoptotic BH3-only protein regulated by interaction with the myosin V actin motor complex, activated by anoikis.

BH3-only family member HRK (also known as DP5) is involved in apoptosis regulation. Hrk gene expression was found to be restricted to cells and tissues of the central and peripheral nervous systems.

The BH3 interacting-domain death agonist, or BID, gene is a pro-apoptotic member of the Bcl-2 protein family. In response to apoptotic signaling, BID interacts with another Bcl-2 family protein, Bax, leading to the insertion of Bax into organelle membranes, primarily the outer mitochondrial membrane. Bax is believed to interact with, and induce the opening of the mitochondrial voltage-dependent anion channel, VDAC. Alternatively, growing evidence suggest that activated Bax and/or Bak form an oligomeric pore, MAC in the outer membrane. This results in the release of cytochrome c and other pro-apoptotic factors (such as SMAC/DIABLO) from the mitochondria, often referred to as mitochondrial outer membrane permeabilization, leading to activation of caspases. This defines BID as a direct activator of Bax, a role common to some of the pro-apoptotic Bcl-2 proteins containing only the BH3 domain.

Fas ligand (FasL or CD95L) is a type-II transmembrane protein that belongs to the tumor necrosis factor (TNF) family. Its binding with its receptor induces apoptosis. Fas ligand/receptor interactions play an important role in the regulation of the immune system and the progression of cancer.

The FAS receptor (FasR), also known as apoptosis antigen 1 (APO-1 or APT), cluster of differentiation 95 (CD95) or tumor necrosis factor receptor superfamily member 6 (TNFRSF6) is a protein that in humans is encoded by the TNFRSF6 gene. The Fas receptor is a death receptor on the surface of cells that leads to programmed cell death (apoptosis). It is one of two apoptosis pathways, the other being the mitochondrial pathway. FasR is located on chromosome 10 in humans and 19 in mice. Similar sequences related by evolution (orthologs) are found in most mammals.

Caspases, or cysteine-aspartic proteases or cysteine-dependent aspartate-directed proteases are a family of cysteine proteases that play essential roles in apoptosis (programmed cell death), necrosis, and inflammation. Twelve caspases have been identified in humans. There are two types of apoptotic caspases: initiator (apical) caspases and effector (executioner) caspases. Initiator caspases (e.g., CASP2, CASP8, CASP9, and CASP10) cleave inactive pro-forms of effector caspases, thereby activating them. Effector caspases (e.g., CASP3, CASP6, CASP7) in turn cleave other protein substrates within the cell, to trigger the apoptotic process. The initiation of this cascade reaction is regulated by caspase inhibitors. CASP4 and CASP5, which are overexpressed in some cases of vitiligo and associated autoimmune diseases caused by NALP1 variants, are not currently classified as initiator or effector in MeSH, because they are inflammatory enzymes that, in concert with CASP1, are involved in T-cell maturation. Also contemplated herein are mutants of capspases that retains the pro-apoptotic activity.

Survivin, also called baculoviral inhibitor of apoptosis repeat-containing 5 or BIRC5, is a protein that, in humans, is encoded by the BIRC5 gene. NCBI Reference Sequence: NG_029069.1. Survivin is a member of the inhibitor of apoptosis (IAP) family. The survivin protein functions to inhibit caspase activation, thereby leading to negative regulation of apoptosis or programmed cell death. Contemplated herein are mutants of Survivin that abolish the anti-apopotitc activity.

Death Receptor 4 (DR4) is a protein on the surface of certain cells that binds another protein called TRAIL, which may kill some cancer cells. An increase in the amount or activity of death receptor 4 on cancer cells may kill more cells. Also called DR4, TRAIL receptor 1, TRAIL-R1, and tumor necrosis factor receptor superfamily member 10A.

Tumor necrosis factor receptor superfamily, member 10b, is also known as: DR5, CD262, KILLER, TRICK2, TRICKB, ZTNFR9, TRAILR2, TRICK2A, TRICK2B, TRAIL-R2, KILLER/DR5. The protein is a member of the TNF-receptor superfamily, and contains an intracellular death domain. This receptor can be activated by tumor necrosis factor-related apoptosis inducing ligand (TNFSF10/TRAIL/APO-2L), and transduces apoptosis signal. Mice have a homologous gene, tnfrsf10b, that has been essential in the elucidation of the function of this gene in humans. Studies with FADD-deficient mice suggested that FADD, a death domain containing adaptor protein, is required for the apoptosis mediated by this protein.

A tumor necrosis factor receptor (TNFR), or death receptor, is a trimeric cytokine receptor that binds tumor necrosis factors (TNF). The receptor cooperates with an adaptor protein (such as TRADD, TRAF, RIP), which is important in determining the outcome of the response (e.g. apoptosis, inflammation).

Pro-apoptotic fragments of the pro-apoptotic proteins described herein may also be used. A pro-apoptotic fragment of a pro-apoptotic comprises a portion, but no the full-length sequence of the pro-apoptotic, while retaining the pro-apoptotic activity.

The pro-apoptotic protein or fragments thereof can be a naturally occurring protein which has pro-apoptotic activity, or an active variant of a naturally occurring protein.

As used herein, "active variants" refers to variant peptides which retain pro-apoptotic activity. An active variant differs in amino acid sequence from a reference pro-apoptotic protein but retains pro-apoptotic activity. Active variants of pro-apoptotic proteins or fragments thereof include naturally occurring variants (e.g., allelic forms) and variants which are not known to occur naturally.

In general, pro-apoptotic fragments can contain the functional domain of the pro-apoptotic proteins.

Generally, differences are limited so that the sequences of the reference polypeptide and the active variant are closely similar overall and, in many regions, identical. An active variant of a pro-apoptotic protein or fragment thereof and a reference pro-apoptotic protein or fragment thereof can differ in amino acid sequence by one or more amino acid substitutions, additions, deletions, truncations, fusions or any combination thereof. Preferably, amino acid substitutions are conservative substitutions. A conservative amino acid substitution refers to the replacement of a first amino acid by a second amino acid that has chemical and/or physical properties (e.g., charge, structure, polarity, hydrophobicity/hydrophilicity) which are similar to those of the first amino acid. Conservative substitutions include replacement of one amino acid by another within the following groups: lysine (K), arginine (R) and histidine (H); aspartate (D) and glutamate (E); asparagine (N), glutamine (Q), serine (S), threonine (T), tyrosine (Y), K, R, H, D and E; alanine (A), valine (V), leucine (L), isoleucine (I), proline (P), phenylalanine (F), tryptophan (W), methionine (M), cysteine (C) and glycine (G); F, W and Y; C, S and T.

Active variants of pro-apoptotic proteins or fragments thereof can be prepared using suitable methods, for example, by direct synthesis, mutagenesis (e.g., site directed mutagenesis, scanning mutagenesis) and other methods of recombinant DNA technology. Active variants can be identified and/or selected using a suitable apoptosis assay.

Fusion proteins comprising a pro-apoptotic protein or a fragment thereof are also contemplated. A fusion protein may encompass a polypeptide comprising a pro-apoptotic protein, a fragment thereof, or an active variant thereof as a first moiety, linked via a covalent bond (e.g., a peptide bond) to a second moiety (a fusion partner) not occurring in the pro-apoptotic protein as found in nature. Fusion proteins can be prepared using suitable methods, for example, by direct synthesis, recombinant DNA technology, etc.

The invention also relates to nucleic acids encoding a pro-apoptotic protein, a fragment thereof, or a variant thereof.

### C. Autoantigens.

Optionally, the compositions described herein may comprises an autoantigen. The autoantigen can be encoded by the same polynucleotide, or by a second polynucleotide. Preferably, the autoantigen is present in skin or in hair follicles.

### 4. DELIVERY OF POLYNUCLEOTIDES

Being a negatively charged, high molecular weight molecule, polynucleotides have difficulties in passing spontaneously through the phospholipid cell membranes. Various vectors are hence used in order to permit gene transfer, including viral vectors on the one hand, natural or synthetic chemical and/or biochemical vectors on the other hand.

Viral vectors (retroviruses, adenoviruses, adeno-associated viruses, etc.) are very effective, in particular for passing through the membranes, but present a number of risks, such as pathogenicity, recombination, replication, immunogenicity, and the like. Chemical and/or biochemical vectors enable these risks to be avoided. They are, for example, cations (calcium phosphate, DEAE-dextran, and the like.) which act by forming precipitates with DNA, which precipitates can then be "phagocytosed" by the cells. They can also be liposomes in which the DNA is incorporated and which fuse with the plasma membrane. Synthetic gene transfer vectors are generally cationic lipids or polymers which complex DNA can form therewith a particle carrying positive surface charges. These particles are capable of interacting with the negative charges of the cell membrane and then of crossing the latter. Examples of such vectors can include dioctadecylamidoglycylspermine (DOGS, Transfectam™) or N-[1-(2,3-dioleyloxy)propyl]-N,N,N-trimethylammonium chloride (DOTMA, Lipofectin™). Chimeric proteins have also been developed: they consist of a polycationic portion which condenses the DNA, linked to a ligand which binds to a membrane receptor and gives rise to the complex in the cells by endocytosis. It is thus theoretically possible to "target" a tissue or certain cell populations in order to improve the *in vivo* bioavailability of the transferred gene.

Viral vector can be delivered to a host cell in the form of a recombinant virus. Recombinant viral vector can be packaged into viral coats or capsids by any suitable procedure, for example, by transfecting the recombinant viral vector into a packaging cell line. Any suitable packaging cell line can be used to generate recombinant virus. Suitable packaging lines for retroviruses include derivatives of PA317 cells, ψ-2 cells, CRE cells, CRIP cells, E-86-GP cells, and 293GP cells. Line 293 cells can be used for adenoviruses and adeno-associated viruses. Neuroblastoma cells can be used for herpes simplex virus, e.g. herpes simplex virus type 1. Sometimes, a helper virus (which provides missing proteins for production of new virions) may be needed to produce a recombinant virus described herein.

Nucleic acid molecules encoding a pro-apoptotic protein, or a fragment thereof can also be delivered using a non-viral based nucleic acid delivery system. For example, methods of delivering a nucleic acid to a target cell have been described in U.S. Pat. Nos. 6,413,942, 6,214,804, 5,580,859, 5,589,466, 5,763,270 and 5,693,622.

Nucleic acid molecules described herein can be packaged in liposomes prior to delivery to a subject or to cells, as described in U.S. Pat. Nos. 5,580,859, 5,549,127, 5,264,618, 5,703,055. For a review of the use of liposomes as carriers for delivery of nucleic acids, see, Hug and Sleight (1991) Biochim. Biophys. Acta. 1097:1-17; Straubinger et al. (1983) in Methods of Enzymology Vol. 101, pp. 512-27; de Lima et al. (2003) Current Medicinal Chemistry, Volume 10(14): 1221-31. Representative liposomes include, but not limited to cationic liposomes, optionally coated with polyethylene glycol (PEG) to reduce non-specific binding of serum proteins and to prolong circulation time. See Koning et al., 1999; Nam et al., 1999; and Kirpotin et al., 1997. Temperature-sensitive liposomes can also be used, for example THERMOSOMES™ as disclosed in U.S. Patent No. 6,200,598. The use of vector-liposome complexes has been described in U.S. Patent No. 5,851 ,818. Liposomes can be prepared by any of a variety of techniques that are known in the art. See e.g., Betageri et al., 1993; Gregoriadis, 1993; Janoff, 1999; Lasic & Martin, 1995; Nabel, 1997; and U.S. Patent Nos. 4,235,871 ; 4,551 ,482; 6,197,333; and 6,132,766.

Nucleic acids can also be delivered in cochleate lipid compositions similar to those described by Papahadjopoulos et al. (1975) Biochem. Biophys. Acta. 394:483-491. See also U.S. Pat. Nos. 4,663,161 and 4,871,488. For example, a plasmid vector may be complexed with Lipofectamine 2000. Wang et al. (2005) Mol. Therapy 12(2):314-320.

Biolistic delivery systems employing particulate carriers such as gold and tungsten may also be used to deliver nucleic acids (e.g., recombinant viral vectors and non-viral vectors). The particles are coated with the vector and accelerated to high velocity, generally under reduced pressure, using a gun powder discharge from a "gene gun." See, e.g., U.S. Pat. Nos. 4,945,050, 5,036,006, 5,100,792, 5,179,022, 5,371,015, and 5,478,744.

A wide variety of other methods can be used to deliver the nucleic acids described herein. Such methods include DEAE dextran-mediated transfection, calcium phosphate precipitation, polylysine- or polyornithine-mediated transfection, or precipitation using other insoluble inorganic salts, such as strontium phosphate, aluminum silicates including bentonite and kaolin, chromic oxide, magnesium silicate, talc, and the like. Other useful methods of transfection include electroporation, sonoporation, protoplast fusion, peptoid delivery, or microinjection. See, e.g., Sambrook et al (1989) Molecular Cloning, A Laboratory Manual, Cold Spring Harbor Laboratories, New York, for a discussion of techniques for transforming cells of interest; and Felgner, P. L. (1990) Advanced Drug Delivery Reviews 5:163-87, for a review of delivery systems useful for gene transfer. Exemplary methods of delivering DNA using electroporation are described in U.S. Pat. Nos. 6,132,419; 6,451,002, 6,418,341, 6,233,483, U.S. Patent Publication No. 2002/0146831, and International Publication No. WO 00/45823.

### 5. PHARMACEUTICAL COMPOSITIONS AND METHODS OF TREATMENT

One aspect of the subject technology describes methods to treat hair loss, the use of the polynucleotides described herein in therapy, and the use of the polynucleotides described herein in the manufacture of a medicament for therapy. The term treatment including prophylactic treatment in the absence of a symptom, as well as treatment that alleviated a disease, or symptoms of a disease.

The subject technology provides a pharmaceutical composition comprising a polynucleotide described herein. Optionally, a delivery system for delivering the nucleic acid (e.g., liposomes) may be provided. The delivery system can be co-formulated with the nucleic acid into a pharmaceutical composition, or supplied separately (e.g., in a separate container in a kit). If provided separately, the delivery system may be mixed with the nucleic acid prior to administration.

Polynucleotides disclosed herein are generally formulated in a composition suitable for in vivo administration. Such compositions generally include a carrier that can is acceptable for formulating and administering the agent to a subject. Such acceptable carriers are well known in the art and include, for example, aqueous solutions such as water or physiologically buffered saline or other solvents or vehicles such as glycols, glycerol, oils such as olive oil or injectable organic esters. An acceptable carrier can contain physiologically acceptable compounds that act, for example, to stabilize or to increase the absorption of the conjugate. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of an acceptable carrier, including a physiologically acceptable compound, depends, for example, on the physicochemical characteristics of the therapeutic agent and on the route of administration of the composition, which can be, for example, orally or parenterally such as intravenously, and by injection, intubation, or other such method known in the art. The pharmaceutical composition also can contain a second reagent such as a diagnostic reagent, nutritional substance, toxin, or therapeutic agent, for example, a cancer chemotherapeutic agent.

Polynucleotides described herein can be incorporated within an encapsulating material such as into an oil-in-water emulsion, a microemulsion, micelle, mixed micelle, liposome, microsphere or other polymer matrix (see, for example, Gregoriadis, Liposome Technology, Vol. 1 (CRC Press, Boca Raton, Fla. 1984); Fraley, et al., Trends Biochem. Sci., 6:77 (1981)). Liposomes, for example, which consist of phospholipids or other lipids, are nontoxic, physiologically acceptable and metabolizable carriers that are relatively simple to make and administer. "Stealth" liposomes (see, for example, U.S. Pat. Nos. 5,882,679; 5,395,619; and 5,225,212) are an example of such encapsulating materials particularly useful for preparing a composition useful in a method of the invention, and other "masked" liposomes similarly can be used, such liposomes extending the time that the therapeutic agent remain in the circulation. Cationic liposomes, for example, also can be modified with specific receptors or ligands (Morishita et al., J. Clin. Invest., 91:2580-2585 (1993), which is incorporated herein by reference). In addition, a polynucleotide can be introduced into a cell using, for example, adenovirus-polylysine DNA complexes (see, for example, Michael et al., J. Biol. Chem. 268:6866-6869 (1993)).

In one aspect, the subject technology provides a polynucleotide, wherein said polynucleotide comprises a sequence that encodes a pro-apoptotic protein, or a functional fragment thereof for use in a method of treating Alopecia areata, suitable to be administered to a subject in need thereof, and wherein about 30% to about 60% of the CpG dinucleotides of the polynucleotide are methylated.

In another aspect, the subject technology provides a polynucleotide for use in a method of treating Alopecia areata, suitable to be administered to a subject in need thereof, wherein said polynucleotide comprises a sequence that encodes a pro-apoptotic protein, or a functional fragment thereof, and wherein the methylation level of CpG dinucleotides of the polynucleotide is about 2 fold to about 4 fold higher as compared to the average methylation level of CpG dinucleotides in a wild type Escherichia coli (*E. coli*) genome.

The subject can be an alopecia subject having a history of, or coexisting, allergic disease or autoimmune disease.

Alopecia areata can be normal alopecia areata, alopecia totalis, alopecia universalis, or alopecia ophiasis.

Compositions described herein may also be used to treat white hair, vellus hair formation, or an agent for inhibiting seborrheic scalp or the occurrence of dandruff.

In one aspect, the subject technology provides a polynucleotide, wherein said polynucleotide comprises a sequence that encodes a pro-apoptotic protein, or a functional fragment thereof for use in a method of stimulating growth of a hair shaft from a hair follicle, suitable to be administered to a subject in need thereof, and wherein about 30% to about 60% of the CpG dinucleotides of the polynucleotide are methylated.

In one aspect, the subject technology provides a polynucleotide, wherein said polynucleotide comprises a sequence that encodes a pro-apoptotic protein, or a functional fragment thereof for use in a method of stimulating growth of a hair shaft from a hair follicle, suitable to be administered to a subject in need thereof, and wherein the methylation level of CpG dinucleotides of the polynucleotide is about 2 fold to about 4 fold higher as compared to the average methylation level of CpG dinucleotides in a wild type Escherichia coli (*E. coli*) genome.

In one aspect, the subject technology provides a polynucleotide, wherein said polynucleotide comprises a sequence that encodes a pro-apoptotic protein, or a functional fragment thereof for use in a method of treating hair loss, wherein said hair loss is attributable to an autoimmune disease, suitable to be administered to a subject in need thereof, and wherein about 30% to about 60% of the CpG dinucleotides of the polynucleotide are methylated.

In one aspect, the subject technology provides a polynucleotide, wherein said polynucleotide comprises a sequence that encodes a pro-apoptotic protein, or a functional fragment thereof for use in a method of stimulating growth of a hair shaft from a hair follicle, suitable to be administered to a subject in need thereof, and wherein the methylation level of CpG dinucleotides of the polynucleotide is about 2 fold to about 4 fold higher as compared to the average methylation level of CpG dinucleotides in a wild type Escherichia coli (*E. coli*) genome.

In many instances, hair loss can be attributed to an autoimmune condition. Exemplary autoimmune diseases that could result in hair loss include, e.g., chronic discoid lupus erythematosus, localized scleroderma, pemphigus, pemphigoid, herpes gestationis, linear IgA bullous dermatosis, acquired epidermolysis bullosa, vitiligo, Sutton's nevus, an autoimmune thyroid disease, systemic lupus erythematosus, rheumatoid arthritis, or myasthenia gravis

Androgenetic alopecia can also results in hair loss. Androgenetic alopecia can be in a male or androgenetic alopecia in a female.

An effective amount is administered to a subject in need thereof. An "effective amount" or "therapeutically effective amount" is an amount that is sufficient to achieve the desired therapeutic or prophylactic effect under the conditions of administration, such as an amount sufficient to reduce/ameliorate symptoms of disease.

The route of administration of the composition containing polynucleotides described herein will depend, in part, on the chemical structure of the molecule. Polypeptides and polynucleotides, for example, are not particularly useful when administered orally because they can be degraded in the digestive tract. However, methods for chemically modifying polypeptides, for example, to render them less susceptible to degradation by endogenous proteases or more absorbable through the alimentary tract are disclosed herein or otherwise known in the art (see, for example, Blondelle et al., supra, 1995; Ecker and Crook, supra, 1995). In addition, a polypeptide can be prepared using D-amino acids, or can contain one or more domains based on peptidomimetics, which are organic molecules that mimic the structure of a domain; or based on a peptoid such as a vinylogous peptoid.

A composition as disclosed herein can be administered to an individual by various routes including, for example, orally or parenterally, such as intravenously, intramuscularly, subcutaneously, intraorbitally, intracapsularly, intraperitoneally, intrarectally, intracistemally or by passive or facilitated absorption through the skin using, for example, a skin patch or transdermal iontophoresis, respectively. Furthermore, the composition can be administered by injection, intubation, orally or topically, the latter of which can be passive, for example, by direct application of an ointment, or active, for example, using a nasal spray or inhalant, in which case one component of the composition is an appropriate propellant. A pharmaceutical composition also can be administered to the site of a pathologic condition, for example, to the scalp where hair loss is occurring, or is likely to occur.

Preferred route of administration includes at least one of subcutaneously, intradermally, or transdermally. The compositions can be administered to the hair loss site, e.g., the frontal region or the crown.

The total amount of the polynucleotide to be administered in practicing a method of the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary. In general, the formulation of the composition and the routes and frequency of administration are determined, initially, using Phase I and Phase II clinical trials.

In various embodiments, when the method comprises administering one or more than one immunosuppressant agent(s), the one or more than one immunosuppressant agent(s) can be administered simultaneously, separately or sequentially.

In various embodiments, the one or more than one immunosuppressant agent(s) may be selected from the group consisting of corticosteroids, glucocorticoids. cyclophosphamide, 6-mercaptopurine (6-MP), azathioprine (AZA), methotrexate cyclosporine, mycophenolate mofetil (MMF), mycophenolic acid (MPA), tacrolimus (FK506), sirolimus ([SRL] rapamycin), everolimus (Certican), mizoribine, leflunomide, deoxyspergualin, brequinar, azodicarbonamide, vitamin D analogs, such as MC1288 and bisindolylmaleimide VIII, antilymphocyte globulin, antithymocyte globulin (ATG), anti-CD3 monoclonal antibodies, (Muromonab-CD3, Orthoclone OKT3), anti-interleukin (IL)-2 receptor (anti-CD25) antibodies, (Daclizumab, Zenapax, basiliximab, Simulect), anti-CD52 antibodies, (Alemtuzumab, Campath-1H), anti-CD20 antibodies (Rituximab, Rituxan), antitumor necrosis factor (TNF) reagents (Infliximab, Remicade, Adalimumab, Humira), LFA-1 inhibitors (Efalizumab, Raptiva), and the like.

In one aspect, the subject technology provides a polynucleotide comprising a sequence that encodes a pro-apoptotic protein, or a functional fragment thereof, wherein about 30% to about 60% of the CpG dinucleotides of the polynucleotide are methylated for the use of treating alopecia areata.

In one aspect, the subject technology provides a polynucleotide comprising a sequence that encodes a pro-apoptotic protein, or a functional fragment thereof, wherein the methylation level of CpG dinucleotides of the polynucleotide is about 2 fold to about 4 fold higher as compared to the average methylation level of CpG dinucleotides in a wild type Escherichia coli (*E. coli*) genome for the use of treating alopecia areata.

In one aspect, the subject technology provides a polynucleotide comprising a sequence that encodes a pro-apoptotic protein, or a functional fragment thereof, wherein about 30% to about 60% of the CpG dinucleotides of the polynucleotide are methylated for the use of stimulating growth of a hair shaft from a hair follicle.

In one aspect, the subject technology provides a polynucleotide comprising a sequence that encodes a pro-apoptotic protein, or a functional fragment thereof, wherein the methylation level of CpG dinucleotides of the polynucleotide is about 2 fold to about 4 fold higher as compared to the average methylation level of CpG dinucleotides in a wild type Escherichia coli (*E. coli*) genome for the use of stimulating growth of a hair shaft from a hair follicle.

In one aspect, the subject technology provides a polynucleotide comprising a sequence that encodes a pro-apoptotic protein, or a functional fragment thereof, wherein about 30% to about 60% of the CpG dinucleotides of the polynucleotide are methylated for the use of treating hair loss, wherein said hair loss is attributable to an autoimmune disease.

In one aspect, the subject technology provides a polynucleotide comprising a sequence that encodes a pro-apoptotic protein, or a functional fragment thereof, wherein the methylation level of CpG dinucleotides of the polynucleotide is about 2 fold to about 4 fold higher as compared to the average methylation level of CpG dinucleotides in a wild type Escherichia coli *(E. coli)* genome for the use of treating hair loss, wherein said hair loss is attributable to an autoimmune disease.

The following Examples provide further illustrations of the subject technology and are not intended to limit the scope of the subject technology.

### EXAMPLIFICATION

### EXAMPLE 1. USE OF DNA VACCINE FOR THE TREATMENT OF ALOPECIA AREATA

A plasmid DNA construct encoding a pro-apoptotic protein is made. The pro-apoptotic protein is selected from: Bax, Bak, Bim, Puma, Bad, Bik, Noxa, Bmf, Hrk, Bid, FAS, a caspase mutant, or a survivin mutant. The plasmid is isolated from a bacterial strain that hypermethylates CpG dinucleotides and from a bacterial strain that does not hypermethylate CpG dinucleotides (i.e., hypomethylated). In the case of the hypermethylated DNA construct, the promoter controlling transcription of the gene/cDNA encoding the pro-apoptotic protein is not inactivated by CpG hypermethylation, e.g., the SV40 promoter. In the case of the non-hypermethylated construct the promoter can be any inducible or constitutive promoter, e.g., the CMV or LTR promoter.

Each of the plasmid DNA construct or different combinations of various ratios of the two constructs are then delivered to the skin to treat alopecia areata, e.g., using the C3H/HeJ mouse model. DNA is delivered intradermally either in healthy skin, i.e., with hair, or directly into the diseased area showing hair loss. DNA amounts can range from 1 microgram to 200 microgram or more per treatment, and the treatment is delivered weekly until hair growth is observed. Alternatively, the DNA can be delivered transdermally after packaging into nano or microparticles that promote cell transfection.

In addition, the plasmid DNA construct can also encode an autoantigen present in hair follicles. In this case the DNA can be injected in skin which has no hair follicle or stem cells promoting the formation of hair follicles.

### Appendix: SEQUENCES

### 1. Nucleotide sequence encoding human BAX

atggacgggt ccggggagca gcccagaggc gggggcccca ccagctctga gcagatcatg 60

aagacagggg cccttttgct tcagggtttc atccaggatc gagcagggcg aatggggggg 120

gaggcacccg agctggccct ggacccggtg cctcaggatg cgtccaccaa gaagctgagc 180

gagtgtctca agcgcatcgg ggacgaactg gacagtaaca tggagctgca gaggatgatt 240

gccgccgtgg acacagactc cccccgagag gtctttttcc gagtggcagc tgacatgttt 300

tctgacggca acttcaactg gggccgggtt gtcgcccttt tctactttgc cagcaaactg 360

gtgctcaagg ccctgtgcac caaggtgccg gaactgatca gaaccatcat gggctggaca 420

ttggacttcc tccgggagcg gctgttgggc tggatccaag accagggtgg ttgggacggc 480

ctcctctcct actttgggac gcccacgtgg cagaccgtga ccatctttgt ggcgggagtg 540

ctcaccgcct cgctcaccat ctggaagaag atgggctga 579

## Claims

1. A polynucleotide, wherein the polynucleotide comprises a sequence that encodes a pro-apoptotic protein selected from Bax, Bak, Bim, Puma, Bad, Bik, Noxa, Bmf, Hrk, Bid, FAS, caspase, or a pro-apoptotic fragment thereof, and wherein the methylation level of CpG dinucleotides of the polynucleotide is about 2 fold to about 4 fold higher as compared to the average methylation level of CpG dinucleotides in a wild type Escherichia coli (*E. coli*) genome, or
about 30% to about 60% of the CpG dinucleotides of the polynucleotide are methylated,
for use in a method of stimulating growth of a hair shaft from a hair follicle, of treating alopecia areata, or of treating hair loss attributed to an autoimmune disease.

2. The polynucleotide of claim 1, wherein the use comprises treating alopecia areata.

3. The polynucleotide of claim 2, wherein the alopecia areata comprises alopecia ophiasis, alopecia totalis, or alopecia universalis.

4. The polynucleotide of any one of claims 1-3, wherein hair loss attributed to an autoimmune diseases comprises an autoimmune disease selected from the group consisting of chronic discoid lupus erythematosus, localized scleroderma, pemphigus, pemphigoid, herpes gestationis, linear IgA bullous dermatosis, acquired epidermolysis bullosa, vitiligo, Sutton's nevus, an autoimmune thyroid disease, systemic lupus erythematosus, rheumatoid arthritis, myasthenia gravis, or a combination thereof.

5. The polynucleotide according to any one of claims 1-4, wherein said pro-apoptotic protein is selected from the group consisting of Bax, Bak, Bim, Puma, Bad, Bik, Noxa, Bmf, Hrk, Bid, FAS, and a caspase.

6. The polynucleotide of any one of
claims 1-5, wherein said polynucleotide is suitable to be administered at least one of subcutaneously or intradermally.

7. The polynucleotide of any one of
claims 1-6, wherein said polynucleotide is suitable to be administered to a site of hair loss.

8. The polynucleotide of any one of claims 1-7, wherein said polynucleotide is produced in a bacterium, for example wherein said bacterium comprises an engineered methylase-coding sequence controlled by a constitutive promoter, and wherein said engineered methylase-coding sequence is stably incorporated into the chromosomal DNA of said bacterium.

9. The polynucleotide of claim 8, wherein the bacterium is Escherichia coli (*E*. *coli*)*.*

10. The polynucleotide of claim 8, wherein the methylase is a CpG methylase.

11. A polynucleotide comprising a sequence that encodes a pro-apoptotic protein, or a pro-apoptotic fragment thereof, wherein about 30% to about 60% of the CpG dinucleotides of the polynucleotide are methylated for the use of treating alopecia areata, or
wherein the methylation level of CpG dinucleotides of the polynucleotide is about 2 fold to about 4 fold higher as compared to the average methylation level of CpG dinucleotides in a wild type Escherichia coli (*E. coli*) genome for the use of treating alopecia areata.

12. A polynucleotide comprising a sequence that encodes a pro-apoptotic protein, or a pro-apoptotic fragment thereof, wherein about 30% to about 60% of the CpG dinucleotides of the polynucleotide are methylated for the use of stimulating growth of a hair shaft from a hair follicle, or
wherein the methylation level of CpG dinucleotides of the polynucleotide is about 2 fold to about 4 fold higher as compared to the average methylation level of CpG dinucleotides in a wild type Escherichia coli (*E. coli*) genome for the use of stimulating growth of a hair shaft from a hair follicle.

13. A polynucleotide comprising a sequence that encodes a pro-apoptotic protein, or a pro-apoptotic fragment thereof, wherein about 30% to about 60% of the CpG dinucleotides of the polynucleotide are methylated for the use of treating hair loss attributed to an autoimmune disease, or
wherein the methylation level of CpG dinucleotides of the polynucleotide is about 2 fold to about 4 fold higher as compared to the average methylation level of CpG dinucleotides in a wild type Escherichia coli (*E. coli*) genome for the use of treating hair loss attributed to an autoimmune disease.

## Patentansprüche

1. Polynukleotid, wobei das Polynukleotid eine Sequenz umfasst, die ein pro-apoptotisches Protein, ausgewählt aus Bax, Bak, Bim, Puma, Bad, Bik, Noxa, Bmf, Hrk, Bid, FAS, Caspase, oder ein pro-apoptotisches Fragment davon kodiert, und wobei der Methylierungsgrad von CpG Dinukleotiden des Polynukleotids etwa 2-fach bis etwa 4-fach höher, verglichen mit dem durchschnittlichen Methylierungsgrad von CpG Dinukleotiden in einem Wildtyp Escherichia coli *(E.coli)* Genom ist, oder etwa 30% bis etwa 60% der CpG Dinukleotide der Polynukleotide methyliert sind,
zur Verwendung in einem Verfahren zum Stimulieren des Wachstums eines Haarschafts aus einem Haarfollikel, zum Behandeln von Alopecia areata, oder zum Behandeln von Haarverlust, zurückzuführen auf eine Autoimmunerkrankung.

2. Polynukleotide nach Anspruch 1, wobei die Verwendung Behandeln von Alopecia areata umfasst.

3. Polynukleotid nach Anspruch 2, wobei die Alopecia areata Alopecia ophiasis, Alopecia totalis, oder Alopecia universalis umfasst.

4. Polynukleotid nach einem der Ansprüche 1 - 3, wobei Haarverlust, zurückzuführen auf eine Autoimmunerkrankung, eine Autoimmunerkrankung ausgewählt aus der Gruppe bestehend aus chronischer diskoidaler Lupus erythematodes, lokalisierter Sklerodermie, Pemphigus, Pemphigoid, Herpes gastationis, linearer IgA bullöser Dermatose, erworbener Epidermolysis Bullosa, Vitiligo, Nävus Sutton, einer autoimmunen Schilddrüsenerkrankung, systematischer Lupus erythematodes, rheumatoider Arthritis, Myasthenia gravis, oder eine Kombination davon umfasst.

5. Polynukleotid nach einem der Ansprüche 1 - 4, wobei das proapoptotische Protein ausgewählt ist aus der Gruppe bestehend aus Bax, Bak, Bim, Puma, Bad, Bik, Noxa, Bmf, Hrk, Bid, FAS, und einer Caspase.

6. Polynukleotid nach einem der Ansprüche 1 - 5, wobei das Polynukleotid geeignet ist, mindestens eines aus subkutan oder intradermal verabreicht zu werden.

7. Polynukleotid nach einem der Ansprüche 1 - 6, wobei das Polynukleotid geeignet ist, an einer Stelle des Haarverlusts verabreicht zu werden.

8. Polynukleotid nach einem der Ansprüche 1 - 7, wobei das Polynukleotid in einem Bakterium produziert wird, zum Beispiel wobei das Bakterium eine konstruierte Methylase-kodierende Sequenz, kontrolliert von einem konstitutiven Promoter, umfasst, und wobei die konstruierte Methylase-kodierende Sequenz in die chromosomale DNA des Bakteriums stabil integriert ist.

9. Polynukleotid nach Anspruch 8, wobei das Bakterium Escherichia coli (*E.coli*) ist.

10. Polynukleotid nach Anspruch 8, wobei die Methylase eine CpG Methylase ist.

11. Polynukleotid, umfassend eine Sequenz, die ein pro-apoptotisches Protein, oder ein pro-apoptotisches Fragment davon kodiert, wobei etwa 30% bis etwa 60% der CpG Dinukleotide des Polynukleotids für die Verwendung zum Behandeln von Alopecia areata methyliert sind, oder wobei der Methylierungsgrad von CpG Dinukleotiden des Polynukleotids etwa 2-fach bis etwa 4-fach höher als verglichen mit dem durchschnittlichen Methylierungsgrad von CpG Dinukleotiden in einem Wildtyp Escherichia coli (*E.coli*) Genom für die Verwendung zum Behandeln von Alopecia areata ist.

12. Polynukleotid, umfassend eine Sequenz, die ein pro-apoptotisches Protein, oder ein pro-apoptotisches Fragment davon kodiert, wobei etwa 30% bis etwa 60% der CpG Dinukleotide des Polynukleotids für die Verwendung zum Stimulieren von Wachstum eines Haarschafts aus einem Haarfolikel methyliert sind, oder
wobei der Methylierungsgrad von CpG Dinukleotiden des Polynukleotids etwa 2-fach bis etwa 4-fach höher als verglichen mit dem durchschnittlichen Methylierungsgrad von CpG Dinukleotiden in einem Wildtyp Escherichia coli (*E.coli*) Genom für die Verwendung zum Stimulieren von Wachstum eines Haarschafts aus einem Haarfolikel ist.

13. Polynukleotid, umfassend eine Sequenz, die ein pro-apoptotisches Protein, oder ein pro-apoptotisches Fragment davon kodiert, wobei etwa 30% bis etwa 60% der CpG Dinukleotide des Polynukleotids für die Verwendung zum Behandeln von Haarverlust, zurückzuführen auf eine Autoimmunerkrankung methyliert sind, oder
wobei der Methylierungsgrad von CpG Dinukleotiden des Polynukleotids etwa 2-fach bis etwa 4-fach höher als verglichen mit dem durchschnittlichen Methylierungsgrad von CpG Dinukleotiden in einem Wildtyp Escherichia coli (*E.coli*) Genom für die Verwendung zum Behandeln von Haarverlust, zuzuführen auf eine Autoimmunerkrankung, ist.

## Revendications

1. Un polynucléotide, dans lequel le polynucléotide comprend une séquence codant pour une protéine pro-apoptotique choisie parmi Bax, Bak, Bim, Puma, Bad, Bik, Noxa, Bmf, Hrk, Bid, FAS, une caspase ou un fragment pro-apoptotique de ceux-ci, et dans lequel le niveau de méthylation des dinucléotides CpG du polynucléotide est d'environ 2 fois à environ 4 fois plus élevé par comparaison avec le niveau de méthylation moyen des dinucléotides CpG dans un génome d'Escherichia coli (*E. coli*) de type sauvage, ou environ 30% à environ 60% de celui des dinucléotides CpG du polynucléotide sont méthylés, pour une utilisation dans un procédé de stimulation de la croissance d'une tige pilaire à partir d'un follicule pileux, de traitement de l'alopecia areata ou de traitement de la perte de cheveux attribuée à une maladie auto-immune.

2. Le polynucléotide selon la revendication 1, dans lequel l'utilisation comprend le traitement de l'alopécia areata.

3. Le polynucléotide selon la revendication 2, dans lequel l'alopécia areata comprend l'alopécia ophiasis, l'alopécia totalis ou l'alopécia universalis.

4. Le polynucléotide selon l'une quelconque des revendications 1 à 3, dans lequel la perte de cheveux attribuée à une maladie auto-immune comprend une maladie auto-immune choisie dans le groupe constitué par le lupus érythémateux discoïde chronique, la sclérodermie localisée, le pemphigus, la pemphigoïde, l'herpès gestationis, la dermatose bulleuse à IgA linéaire, l'épidermolyse bulleuse acquise, le vitiligo, le naevus de Sutton, une maladie thyroïdienne auto-immune, le lupus érythémateux disséminé, la polyarthrite rhumatoïde, la myasthénie grave ou une combinaison de ceux-ci.

5. Le polynucléotide selon l'une quelconque des revendications 1 à 4, dans lequel ladite protéine pro-apoptotique est choisie dans le groupe constitué de Bax, Bak, Bim, Puma, Bad, Bik, Noxa, Bmf, Hrk, Bid, FAS et une caspase.

6. Le polynucléotide selon l'une quelconque des revendications 1 à 5, dans lequel ledit polynucléotide convient pour une administration au moins par voie sous-cutanée ou intradermique.

7. Le polynucléotide selon l'une quelconque des revendications 1 à 6, dans lequel ledit polynucléotide convient pour une administration à une zone de perte de cheveux.

8. Le polynucléotide selon l'une quelconque des revendications 1 à 7, dans lequel ledit polynucléotide est produit dans une bactérie, par exemple dans lequel ladite bactérie comprend une séquence modifiée artificiellement codant pour la méthylase, contrôlée par un promoteur constitutif, et dans lequel ladite séquence modifiée artificiellement codant pour la méthylase est incorporée de façon stable dans l'ADN chromosomique de ladite bactérie.

9. Le polynucléotide selon la revendication 8, dans lequel la bactérie est Escherichia coli (*E. coli*).

10. Le polynucléotide selon la revendication 8, dans lequel la méthylase est une méthylase CpG.

11. Un polynucléotide comprenant une séquence codant pour une protéine pro-apoptotique, ou un fragment pro-apoptotique de celle-ci, dans lequel environ 30% à environ 60% des dinucléotides CpG du polynucléotide sont méthylés, pour une utilisation dans le traitement de l'alopécia areata, ou dans lequel le niveau de méthylation des dinucléotides CpG du polynucléotide est d'environ 2 fois à environ 4 fois plus élevé par comparaison avec le niveau de méthylation moyen des dinucléotides CpG dans un génome d'Escherichia coli (*E. coli*) de type sauvage, pour une utilisation dans le traitement de l'alopécia areata.

12. Un polynucléotide comprenant une séquence codant pour une protéine pro-apoptotique, ou un fragment pro-apoptotique de celle-ci, dans lequel environ 30% à environ 60% des dinucléotides CpG du polynucléotide sont méthylés, pour une utilisation de stimulation de la croissance d'une tige capillaire à partir d'un follicule pileux, ou dans lequel le niveau de méthylation des dinucléotides CpG du polynucléotide est environ 2 fois à environ 4 fois plus élevé par comparaison avec le niveau moyen de méthylation des dinucléotides CpG dans un génome de Escherichia coli (*E. coli*) de type sauvage, pour une utilisation de stimulation de la croissance d'une tige capillaire à partir d'un follicule pileux.

13. Un polynucléotide comprenant une séquence codant pour une protéine pro-apoptotique, ou un fragment pro-apoptotique de celle-ci, dans lequel environ 30% à environ 60% des dinucléotides CpG du polynucléotide sont méthylés, pour une utilisation de traitement de la perte de cheveux attribuée à un maladie auto-immune, ou dans laquelle le niveau de méthylation des dinucléotides CpG du polynucléotide est environ 2 fois à environ 4 fois plus élevé par comparaison avec le niveau moyen de méthylation des dinucléotides CpG dans un génome d'Escherichia coli (*E. coli*) de type sauvage, pour une utilisation de traitement de la perte de cheveux attribuée à un maladie auto-immune.
